# EUROPÄISCHE PATENTANMELDUNG

(11) **EP 4 292 635 A2**
(43) Veröffentlichungstag der Anmeldung: **20.12.2023**
(21) Anmeldenummer: 23173723.0
(22) Anmeldetag: 16.05.2023
(51) Int. Cl.: A61M 25/00, A61B 17/34, A61M 25/02

(54) **KATHETERSET ZUM AUSBILDEN UNTERSCHIEDLICHER KATHETERANORDNUNGEN FÜR EINE PERIPHERE REGIONALANÄSTHESIE**

(30) Priorität: 23.05.2022 DE 102022205129
(71) Anmelder: B. Braun Melsungen AG, 34212 Melsungen (DE)
(72) Erfinder: Blum, Iris, 34277 Fuldabrück (DE); Weiß, André, 34302 Guxhagen (DE); Bode, Nicky, 37308 Schimberg (DE); Felmeden, Jacqueline, 34242 Melsungen (DE)
(74) Vertreter: Patentanwälte Ruff, Wilhelm, Beier, Dauster & Partner mbB

(57) **Zusammenfassung**

Katheterset (1) zum Ausbilden unterschiedlicher Katheteranordnungen (10, 20, 30) für eine periphere Regionalanästhesie, aufweisend ein Kapillar (100) mit Kapillarrohr (101) und einem Kapillaransatz (102), welcher an ein proximales Ende (103) des Kapillarrohrs angefügt ist, eine Kanüle (200) mit einem Kanülenrohr (201), welches in das Kapillarrohr eingeschoben oder einschiebbar ist, und mit einen Kanülenansatz (202), welcher an ein proximales Ende (203) des Kanülenrohrs angefügt und mit dem Kapillaransatz lösbar verbunden oder verbindbar ist, einen Katheterschlauch (300), welcher in distaler Richtung durch das Kapillarrohr vorschiebbar ist, eine Katheterkupplung (400), welche zur Befestigung an einem proximalen Ende des Katheterschlauchs eingerichtet ist, und eine Fluidzuleitung mit einem Schlauch und wenigstens einem Fluidkonnektor, welcher an ein distales Ende des Schlauchs angefügt und mit dem Kapillaransatz lösbar verbindbar ist, wobei das Katheterset zum wahlweisen Ausbilden wenigstens einer ersten Katheteranordnung und einer zweiten Katheteranordnung eingerichtet ist.

## Beschreibung

Die Erfindung betrifft ein Katheterset zum Ausbilden unterschiedlicher Katheteranordnungen für eine periphere Regionalanästhesie.

Bei einer peripheren Regionalanästhesie wird die Weiterleitung von Schmerzimpulsen eines einzelnen Nervs gezielt blockiert. In diesem Zusammenhang wird auch von einer Nervenblockade oder einem Nervenblock gesprochen. Die Blockade erfolgt durch die Verabreichung eines Anästhetikums in unmittelbarer Nähe des betreffenden Nervs.

Katheteranordnungen für die periphere Regionalanästhesie sind seit geraumer Zeit bekannt und werden unter Verwendung sogenannter Kathetersets am Patienten angelegt. Dabei sind unterschiedliche Vorgehensweisen zur Anlage bekannt. Diese unterschiedlichen Vorgehensweisen, auch als Katheterlagen bezeichnet, kommen situationsadäquat oder abhängig von der Präferenz des ausführenden Anästhesisten zum Einsatz. Bekannte Katheterlagen sind beispielsweise geläufig unter den Bezeichnungen "Braunülen-Technik", "Katheter-durch-Nadel-Technik" und "Katheter-über-Nadel-Technik".

Für unterschiedliche Katheterlagen sind unterschiedliche Kathetersets bekannt. Die bekannten Kathetersets sind jeweils aus einer Anzahl von Set-Komponenten zusammengestellt, beispielsweise einer Kanüle, einer Kapillare, einer Fluidzuleitung etc. Für die besagten unterschiedlichen Katheterlagen bietet die Anmelderin am Markt Kathetersets an unter der Bezeichnung Contiplex^{®} D, Contiplex^{®} S, Contiplex^{®} Tuohy sowie Contiplex^{®} C. Die Set-Komponenten der bekannten Kathetersets sind hinsichtlich ihrer gegenständlichen und funktionellen Merkmale spezifisch auf die jeweilige Katheterlage abgestimmt. Jedes der Kathetersets ist jeweils für eine bestimmte Katheterlage und damit zur Ausbildung einer spezifischen Katheteranordnung eingerichtet.

Aufgabe der Erfindung ist es, ein Katheterset der eingangs genannten Art bereitzustellen, welches Vorteile gegenüber dem Stand der Technik bietet.

Diese Aufgabe wird durch das Bereitstellen eines Kathetersets mit den Merkmalen des Anspruchs 1 gelöst. Vorteilhafte Ausgestaltungen sind Gegenstand der Unteransprüche. Deren Wortlaut wird durch Bezugnahme zum Bestandteil der Beschreibung gemacht.

Das erfindungsgemäße Katheterset weist ein Kapillar, eine Kanüle, einen Katheterschlauch, eine Katheterkupplung und eine Fluidzuleitung auf. Die vorgenannten Komponenten des Kathetersets können wahlweise zum Ausbilden einer ersten Katheteranordnung und einer zweiten Katheteranordnung verwendet werden. Die erste Katheteranordnung ist einer ersten Katheterlage zugeordnet. Die zweite Katheteranordnung ist einer unterschiedlichen zweiten Katheterlage zugeordnet. Hierdurch ermöglicht das erfindungsgemäße Katheterset eine besonders vielseitige und vereinfachte Verwendbarkeit. Der ausführende Anästhesist kann unter Verwendung des erfindungsgemäßen Kathetersets kurzfristig, situationsadäquat und/oder gemäß der eigenen Präferenz entscheiden, welche Katheterlage zum Einsatz kommen soll. Zudem erlaubt das erfindungsgemäße Katheterset eine vereinfachte Herstellung, Logistik und Lagerhaltung. Um zwei unterschiedliche Katheterlagen zu ermöglichen, müssen bislang zwei unterschiedliche Kathetersets hergestellt, kommissioniert und beim Endanwender vorgehalten werden. Mit dem erfindungsgemäßen Katheterset ergeben sich diesbezügliche Vereinfachungen und folglich Kosteneinsparungen. Das Kapillar weist ein Kapillarrohr und einen Kapillaransatz auf. Der Kapillaransatz ist an ein proximales Ende des Kapillarrohrs angefügt. Hierfür geeignete Fügeverfahren sind dem Fachmann bekannt. Die Kanüle weist ein Kanülenrohr und einen Kanülenansatz auf. Der Kanülenansatz ist an ein proximales Ende des Kanülenrohrs angefügt. Hierfür geeignete Fügeverfahren sind dem Fachmann bekannt. Bei einer Ausgestaltung des Kathetersets ist in einem Auslieferungszustand das Kanülenrohr über den Kapillaransatz in das Kapillarrohr eingeschoben und der Kanülenansatz und der Kapillaransatz sind lösbar miteinander verbunden. Dies beispielsweise mittels einer form- und/oder kraftschlüssigen Verbindung. Der Kapillaransatz und der Kanülenansatz sind diesbezüglich aufeinander abgestimmt. Die Kanüle dient insbesondere der Punktion des Patienten. Die Punktion erfolgt in einem mit dem Kapillar zusammengefügten Zustand, so dass das Kanülenrohr gemeinsam mit dem auf dem Kanülenrohr abgestützten Kapillarrohr in das Körpergewebe des Patienten vorgeschoben wird. Bei einer weiteren Ausgestaltung ist im Auslieferungszustand die Kanüle örtlich getrennt von dem Kapillar. Das Kanülenrohr ist in diesem Fall über den Kapillaransatz in das Kapillarrohr einschiebbar. In diesem Fall sind der Kanülenansatz und der Kapillaransatz lösbar miteinander verbindbar. Das Kapillarrohr und das Kanülenrohr sind maßlich aufeinander abgestimmt. Insbesondere ist ein Außendurchmesser des Kanülenrohrs kleiner als ein Innendurchmesser des Kapillarrohrs. Vorzugsweise ist eine axiale Länge des Kanülenrohrs länger als eine axiale Länge des Kapillarrohrs. Der Katheterschlauch ist in distaler Richtung durch das Kapillarrohr vorschiebbar. Zu diesem Zweck wird ein distales Schlauchende durch den Kapillaransatz in das Kapillarrohr eingeführt und distal entlang des Kapillarrohrs vorgeschoben. Der Katheterschlauch und das Kapillarrohr sind maßlich aufeinander abgestimmt. Insbesondere ist ein Außendurchmesser des Katheterschlauchs kleiner als ein Innendurchmesser des Kapillarrohrs. Die Katheterkupplung ist zur Befestigung an einem proximalen Ende des Katheterschlauchs eingerichtet. Katheterkupplungen sind dem Fachmann grundsätzlich bekannt und dienen zur fluidleitenden Verbindung eines Katheterschlauchs mit weiteren fluidleitenden Komponenten. Eine für diesen Zweck geeignete Katheterkupplung wird seitens der Anmelderin unter der Bezeichnung Perifix^{®} am Markt angeboten. Die Fluidzuleitung weist einen Schlauch und wenigstens einen Fluidkonnektor auf. Der wenigstens eine Fluidkonnektor ist am distalen Ende des Schlauchs angefügt und mit dem Kapillaransatz lösbar verbindbar. Die Fluidzuleitung kann auch als extension line bezeichnet werden. Vorzugsweise weist die Fluidzuleitung einen weiteren Fluidkonnektor auf, welcher an ein proximales Ende des Schlauchs angefügt ist.

Die erste Katheteranordnung weist den Katheterschlauch und die am proximalen Ende desselben befestigte Katheterkupplung auf. Vorzugsweise besteht die erste Katheteranordnung aus dem Katheterschlauch und der an demselben befestigten Katheterkupplung. Unter Verwendung der ersten Katheteranordnung erfolgt die Abgabe des Anästhetikums über den Katheterschlauch. Die zweite Katheteranordnung weist das Kapillar und die Fluidzuleitung auf. Letztere ist mittels des Fluidkonnektors mit dem Kapillaransatz verbunden. Vorzugsweise besteht die zweite Katheteranordnung aus dem Kapillar und der Fluidzuleitung. Unter Verwendung der zweiten Katheteranordnung erfolgt die Abgabe des Anästhetikums über das Kapillarrohr. Eine Zuleitung zu dem Kapillarrohr erfolgt mittels der Fluidzuleitung. Zum Ausbilden der ersten Katheteranordnung können die Komponenten des Kathetersets wie folgt verwendet werden. In einem ersten Schritt wird die Kanüle mitsamt des Kapillars in das Körpergewebe eingestochen. In einem weiteren Schritt wird die Kanüle entfernt. Hierzu wird der Kanülenansatz von dem Kapillaransatz gelöst, das in dem Kapillarrohr befindliche Kanülenrohr wird in proximaler Richtung herausgezogen. In einem weiteren Schritt wird der Katheterschlauch angebracht. Hierfür wird dessen distales Ende durch den Kapillaransatz in das Kapillarrohr eingeführt und distal vorgeschoben. In einem weiteren Schritt wird das Kapillar entfernt. Hierfür wird das Kapillarrohr gemeinsam mit dem Kapillaransatz proximal von dem Katheterschlauch abgezogen. In einem weiteren Schritt wird die Katheterkupplung an dem proximalen Ende des Katheterschlauchs befestigt. Zum Ausbilden der zweiten Katheteranordnung können die Komponenten des Kathetersets wie folgt verwendet werden. In einem ersten Schritt wird die Kanüle mitsamt des Kapillars in das Körpergewebe des Patienten eingestochen. In einem weiteren Schritt wird die Kanüle entfernt. Hierzu wird auf das bereits Gesagte verwiesen. In einem weiteren Schritt wird die Fluidzuleitung an dem Kapillar angebracht. Hierfür wird der Fluidkonnektor mit dem Kapillaransatz verbunden.

In Ausgestaltung der Erfindung weist das Katheterset eine Fixierkomponente auf. Die Fixierkomponente ist an dem Kapillaransatz befestigbar und zur axialen Fixierung des in das Kapillarrohr eingeschobenen Katheterschlauchs eingerichtet. Bei dieser Ausgestaltung der Erfindung ist das Katheterset zum wahlweisen Ausbilden einer (zusätzlichen) dritten Katheteranordnung eingerichtet. Die dritte Katheteranordnung weist das Kapillar, den Katheterschlauch und die Fixierkomponente auf. Zudem weist die dritte Katheteranordnung die an dem proximalen Ende des Katheterschlauchs befestigte Katheterkupplung auf. Vorzugsweise besteht die dritte Katheteranordnung aus dem Kapillar, dem Katheterschlauch, der Fixierkomponente und der Katheterkupplung. Die Fixierkomponente ist in ausgebildetem Zustand der dritten Katheteranordnung an dem Kapillaransatz befestigt und der Katheterschlauch ist zur Abgabe des Anästhetikums in distaler Richtung durch das Kapillarrohr vorgeschoben sowie mittels der Fixierkomponente relativ zu dem Kapillar axial fixiert. Die dritte Katheteranordnung erlaubt es, einen axialen Überstand des distalen Endes des Katheterschlauchs über das distale Ende des Kapillarrohrs anzupassen. Zwecks Anpassung des axialen Überstands kann der Katheterschlauch relativ zu dem Kapillar mehr oder weniger weit axial vorgeschoben oder zurückgezogen werden. Nach erfolgter Anpassung des Überstands wird die relative Position des Katheterschlauchs gegenüber dem Kapillar mittels der Fixierkomponente fixiert. Bei einer Ausgestaltung ist die Fixierkomponente an einem proximalen Ende des Kapillaransatzes befestigt. Bei einer weiteren Ausgestaltung ist die Fixierkomponente in eine Aufnahmeaussparung des Kapillaransatzes wenigstens abschnittsweise eingesetzt. Zur Fixierung wirkt die Fixierkomponente kraft- und/oder formschlüssig mit dem Katheterschlauch zusammen. Hierfür kann die Fixierkomponente einen Rast-, Klemm-, Exzenter- oder sonstigen Fixiermechanismus aufweisen. Zum Ausbilden der dritten Katheteranordnung können die Komponenten des Kathetersets wie folgt verwendet werden. In einem ersten Schritt wird das auf der Kanüle abgestützte Kapillar angebracht. Hierfür wird das Körpergewebe des Patienten im Bereich des zu blockierenden Nervs punktiert. Das Kanülenrohr wird hierbei mitsamt des Kapillarrohrs distal in das Körpergewebe vorgeschoben. In einem weiteren Schritt wird die Kanüle entfernt. Das diesbezüglich zu der ersten und/oder zweiten Katheteranordnung Gesagte gilt vorliegend mutatis mutandis. In einem weiteren Schritt wird die Fixierkomponente an dem Kapillar angebracht. Hierfür wird die Fixierkomponente an dem Kapillaransatz befestigt. Die Fixierkomponente und der Kapillaransatz sind dementsprechend aufeinander abgestimmt. In einem weiteren Schritt wird der Katheterschlauch angebracht. Hierfür wird dessen distales Ende durch die Fixierkomponente und den Kapillaransatz in das Kapillarrohr eingeführt und distal entlang des Kapillarrohrs vorgeschoben. In einem weiteren Schritt wird der Katheterschlauch relativ zu dem Kapillar positioniert. Hierfür wird dessen distales Ende bis zu einer gewünschten Position in Bezug auf das distale Ende des Kapillarrohrs vorgeschoben und/oder zurückgezogen. In einem weiteren Schritt wird der Katheterschlauch fixiert. Hierfür wird die Fixierkomponente betätigt und der Katheterschlauch wird unter Einwirkung der Fixierkomponente relativ zu dem Kapillar axial festgelegt. In einem weiteren Schritt wird die Katheterkupplung an dem proximalen Ende des Katheterschlauchs befestigt.

In weiterer Ausgestaltung der Erfindung ist das Kapillarrohr biegeflexibel ausgebildet. Die biegeflexiblen Eigenschaften des Kapillarrohrs sind insbesondere im Hinblick auf die zweite Katheteranordnung vorteilhaft. Bei der zweiten Katheteranordnung erfolgt die Abgabe des Anästhetikums über das Kapillarrohr. Das Kapillarrohr verbleibt dementsprechend während der Dauer der, insbesondere kontinuierlichen, peripheren Regionalanästhesie angelegt. Durch die biegeflexible Ausbildung des Kapillarrohrs wird insbesondere ein verbesserter Komfort des Patienten ermöglicht.

In weiterer Ausgestaltung der Erfindung ist das Kapillarrohr aus Polyurethan gefertigt. Die Erfinder haben erkannt, dass Polyurethan für die Fertigung des Kapillarrohrs besondere Vorteile bietet.

In weiterer Ausgestaltung der Erfindung weist der Katheterschlauch einen herausziehbaren Mandrin auf. Der Mandrin erleichtert das Einführen und Vorschieben des Katheterschlauchs. Der Mandrin ist in proximaler Richtung aus dem Katheterschlauch herausziehbar. Diese Ausgestaltung ist insbesondere dann vorteilhaft, wenn der Katheterschlauch einen vergleichsweise kleinen Durchmesser aufweist und dementsprechend biegeschlaff ist. Der Mandrin bewirkt eine mechanische Stabilisierung des Katheterschlauchs.

In weiterer Ausgestaltung der Erfindung sind mehrere zur ultraschallbasierten Ortung eingerichtete Sensoren vorhanden und an unterschiedlichen Komponenten des Kathetersets angebracht. Sensoren zur ultraschallbasierten Ortung von medizinischen Invasivkomponenten sind dem Fachmann bekannt. Bei dieser Ausgestaltung sind unterschiedliche Komponenten des Kathetersets, beispielsweise das Kapillar, die Kanüle und/oder der Katheterschlauch, mit einem solchen Sensor ausgestattet. Die entsprechende Komponente wird dadurch "ultraschallsichtbar". Dies erlaubt eine ultraschallbasierte Ortung und/oder Navigation der betreffenden Komponente bei der Anlage am Patienten.

In weiterer Ausgestaltung der Erfindung ist ein erster Sensor in das distale Ende des Kapillarrohrs integriert. Alternativ oder zusätzlich ist ein zweiter Sensor in das distale Ende des Kanülenrohrs integriert. Alternativ oder zusätzlich ist ein dritter Sensor in das distale Ende des Katheterschlauchs integriert. Sofern der Katheterschlauch einen herausziehbaren Mandrin aufweist, kann letzterer mit dem dritten Sensor oder einem weiteren vierten Sensor versehen sein. Durch die Ausstattung der jeweiligen Komponente mit einem der besagten Sensoren kann eine ultraschallbasierte Positionskontrolle und/oder Navigation erfolgen.

In weiterer Ausgestaltung der Erfindung weist die Fixierkomponente ein Betätigungselement und ein formnachgiebiges Klemmelement auf, wobei das Klemmelement - im ausgebildeten Zustand der dritten Katheteranordnung - in einer Aufnahmeaussparung des Kapillaransatzes aufgenommen ist und ein koaxial zu dem Kapillarrohr ausgerichtetes Lumen aufweist, durch welches der Katheterschlauch längserstreckt ist. Das Betätigungselement ist relativ zu dem Kapillaransatz beweglich an dem Kapillaransatz gelagert. Zudem ist das Klemmelement kraft- und/oder bewegungsübertragend mit dem Klemmelement wirkverbunden. Das Klemmelement ist mittels einer Bewegung des Betätigungselements elastisch deformierbar. Die elastische Deformation erfolgt zwischen einer Klemmkonfiguration und einer Freigabekonfiguration. In der Klemmkonfiguration ist das Klemmelement derart elastisch deformiert, dass das Lumen radial verengt und eine axiale Beweglichkeit des Katheterschlauchs in dem Lumen reibschlüssig fixiert ist. In der Freigabekonfiguration ist das Klemmelement nicht oder weniger stark elastisch deformiert, so dass die radiale Verengung des Lumens und damit auch der Reibschluss aufgehoben sind und die axiale Beweglichkeit des Katheterschlauchs freigegeben ist. Die mit dieser Gestaltung der Fixierkomponente einhergehenden gegenständlichen und funktionellen Merkmale sind besonders vorteilhaft.

Die Erfindung betrifft zudem ein Verfahren zum Ausbilden einer ersten Katheteranordnung. Die erste Katheteranordnung ist zur Verwendung bei einer, insbesondere kontinuierlichen, peripheren Regionalanästhesie eingerichtet. Die erste Katheteranordnung ist einer ersten Katheterlage zugeordnet. Das Verfahren zum Ausbilden der ersten Katheteranordnung weist die folgenden Schritte auf: Bereitstellen eines Kathetersets gemäß der vorhergehenden Beschreibung; Entfernen der Kanüle, wobei der Kanülenansatz von dem Kapillaransatz gelöst und das in dem Kapillarrohr vorgeschobene Kanülenrohr in proximaler Richtung aus dem Kapillarrohr herausgezogen wird; Anbringen des Katheterschlauchs, wobei dessen distales Ende in den Kapillaransatz eingeführt und in distaler Richtung entlang des Kapillarrohrs vorgeschoben wird; Entfernen des Kapillars, wobei das Kapillarrohr in proximaler Richtung von dem Katheterschlauch abgezogen wird; Befestigen der Katheterkupplung an dem proximalen Ende des Katheterschlauchs.

Die Erfindung betrifft zudem ein Verfahren zum Ausbilden einer zweiten Katheteranordnung. Die zweite Katheteranordnung ist zur Verwendung bei einer, insbesondere kontinuierlichen, peripheren Regionalanästhesie vorgesehen. Die zweite Katheteranordnung ist einer zweiten Katheterlage zugeordnet. Das Verfahren zum Ausbilden der zweiten Katheteranordnung weist die Schritte auf: Bereitstellen eines Kathetersets gemäß der vorhergehenden Beschreibung; Entfernen der Kanüle, wobei der Kanülenansatz von dem Kapillaransatz gelöst und das in dem Kapillarrohr vorgeschobene Kanülenrohr in proximaler Richtung aus dem Kapillarrohr herausausgezogen wird; Anbringen der Fluidzuleitung, wobei deren Fluidkonnektor mit dem Kapillaransatz verbunden wird. Zum Verbinden des Fluidkonnektors mit dem Kapillaransatz kann der Fluidkonnektor mit dem Kapillaransatz verschraubt, zusammengesteckt oder auf sonstige geeignete Weise kraft- und/oder formschlüssig lösbar verbunden werden.

Die Erfindung betrifft zudem ein Verfahren zum Ausbilden einer dritten Katheteranordnung. Auch die dritte Katheteranordnung ist zur Verwendung bei einer, insbesondere kontinuierlichen, peripheren Regionalanästhesie eingerichtet. Die dritte Katheteranordnung ist einer dritten Katheterlage zugeordnet. Das Verfahren zum Ausbilden der dritten Katheteranordnung weist die Schritte auf: Bereitstellen eines Kathetersets gemäß der vorhergehenden Beschreibung; Entfernen der Kanüle, wobei der Kanülenansatz von dem Kapillaransatz gelöst und das in dem Kapillarrohr vorgeschobene Kanülenrohr in proximaler Richtung aus dem Kapillarrohr herausgezogen wird; Anbringen der Fixierkomponente an dem Kapillar, wobei die Fixierkomponente an dem Kapillaransatz befestigt wird; Anbringen des Katheterschlauchs, wobei dessen distales Ende in den Kapillaransatz eingeführt und in distaler Richtung entlang des Kapillarrohrs vorgeschoben wird; Positionieren des Katheterschlauchs, wobei dessen distales Ende bis zu einer gewünschten Position in Bezug auf das distale Ende des Kapillarrohrs vorgeschoben wird; Fixieren des Katheterschlauchs, wobei die Fixierkomponente betätigt und der Katheterschlauch unter Einwirkung der Fixierkomponente relativ zu dem Kapillar axial fixiert wird; Befestigen der Katheterkupplung an dem proximalen Ende des Katheterschlauchs. Die Fixierkomponente wird kraft- und/oder formschlüssig an dem Kapillaransatz befestigt. Sofern die Fixierkomponente ein Betätigungselement und ein Klemmelement aufweist, wird das Betätigungselement vorzugsweise mit dem Kapillaransatz verschraubt. Das Klemmelement wird vorzugsweise in eine/die Aufnahmeaussparung des Kapillaransatzes eingesetzt. Zum Anbringen des Katheterschlauchs wird dessen distales Ende durch die Fixierkomponente und den Kapillaransatz in das Kapillarrohr eingeführt. Der Katheterschlauch wird positioniert, indem er bis zum Erreichen eines gewünschten axialen Überstands seines distalen Endes über das distale Ende des Kapillarrohrs vorgeschoben und/oder zurückgezogen wird. Sofern die Fixierkomponente ein Betätigungselement und ein Klemmelement gemäß der oben beschriebenen Ausgestaltung des Kathetersets aufweist, wird das Klemmelement zum Fixieren des Katheterschlauchs ausgehend von der Freigabe- in die Klemmkonfiguration überführt. Dies erfolgt mittels einer Betätigung des Betätigungselements. Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt in schematisch stark vereinfachter Darstellung eine Ausführungsform eines erfindungsgemäßen Kathetersets mit einem Kapillar, einer Kanüle, einem Katheterschlauch, einer Katheterkupplung, einer Fluidzuleitung und einer Fixierkomponente,
- Fig. 2: in schematischer Detaildarstellung das Kapillar des Kathetersets nach Fig. 1,
- Fig. 3: in schematischer Detaildarstellung die Kanüle des Kathetersets nach Fig. 1,
- Fig. 4: in schematisch stark vereinfachter Darstellung eine erste Katheteranordnung, wobei die erste Katheteranordnung unter Verwendung des Kathetersets nach Fig. 1 ausgebildet ist,
- Fig. 5: in schematisch stark vereinfachter Darstellung eine unter Verwendung des Kathetersets nach Fig. 1 ausgebildete zweite Katheteranordnung,
- Fig. 6: in schematisch stark vereinfachter Darstellung eine unter Verwendung des Kathetersets nach Fig. 1 ausgebildete dritte Katheteranordnung,
- Fig. 7: die dritte Katheteranordnung nach Fig. 6 in einer alternativen schematischen Darstellung,
- Fig. 8: in teilweise abgeschnittener schematischer Längsschnittdarstellung entlang eines Schnitts A-A die dritte Katheteranordnung nach Fig. 7,
- Fig. 9: in schematischer Blockdarstellung eine Ausführungsform eines erfindungsgemäßen Verfahrens zum Ausbilden der ersten Katheteranordnung nach Fig. 4 unter Verwendung des Kathetersets nach Fig. 1,
- Fig. 10: in schematischer Blockdarstellung eine Ausführungsform eines erfindungsgemäßen Verfahrens zum Ausbilden der zweiten Katheteranordnung nach Fig. 5 unter Verwendung des Kathetersets nach Fig. 1 und
- Fig. 11: in schematischer Blockdarstellung eine Ausführungsform eines erfindungsgemäßen Verfahrens zum Ausbilden der dritten Katheteranordnung nach den Fig. 6 bis 8 unter Verwendung des Kathetersets nach Fig. 1.

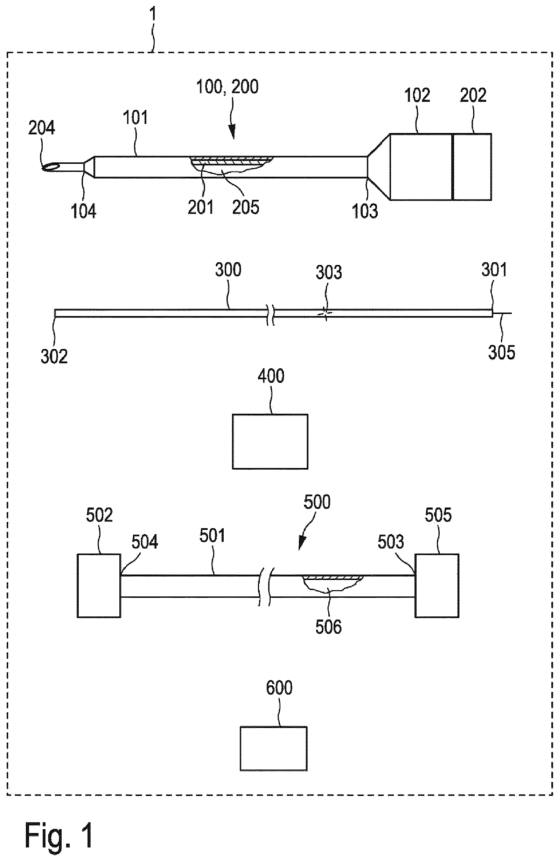

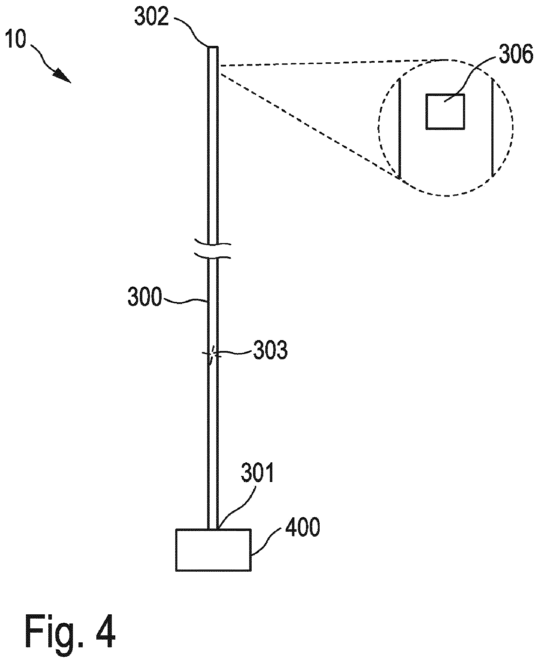

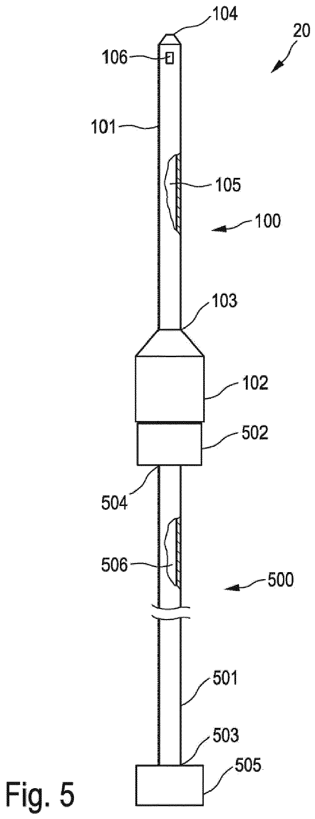

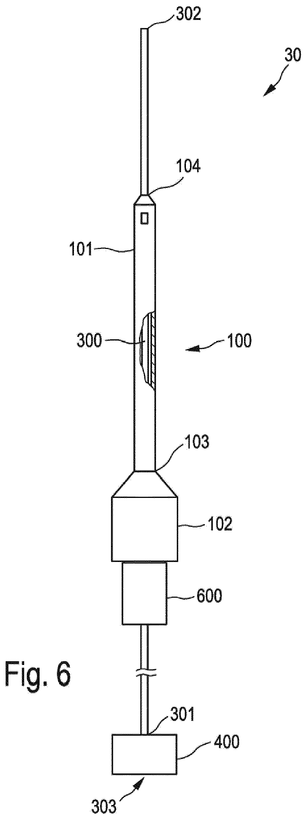

Gemäß Fig. 1 ist ein Katheterset 1 zur Verwendung bei einer peripheren Regionalanästhesie vorgesehen. Das Katheterset 1 ist zum wahlweisen Ausbilden unterschiedlicher Katheteranordnungen 10, 20, 30 eingerichtet. Im Speziellen ist das Katheterset 1 zum Ausbilden einer ersten Katheteranordnung 10 (Fig. 4), einer zweiten Katheteranordnung (Fig. 5) und einer dritten Katheteranordnung (Fig. 6 bis 8) eingerichtet. Zum Ausbilden der unterschiedlichen Katheteranordnungen 10, 20, 30 werden die - noch näher bezeichneten - Komponenten des Kathetersets 1 in unterschiedlicher Weise miteinander kombiniert.

Das Katheterset 1 weist ein Kapillar 100, eine Kanüle 200, einen Katheterschlauch 300, eine Katheterkupplung 400 und eine Fluidzuleitung 500 auf.

Bei der gezeigten Ausführungsform weist das Katheterset 1 zudem eine Fixierkomponente 600 auf. Die Fixierkomponente 600 ist optional. Bei einer in den Figuren nicht gesondert gezeigten Ausführungsform weist das Katheterset keine Fixierkomponente auf.

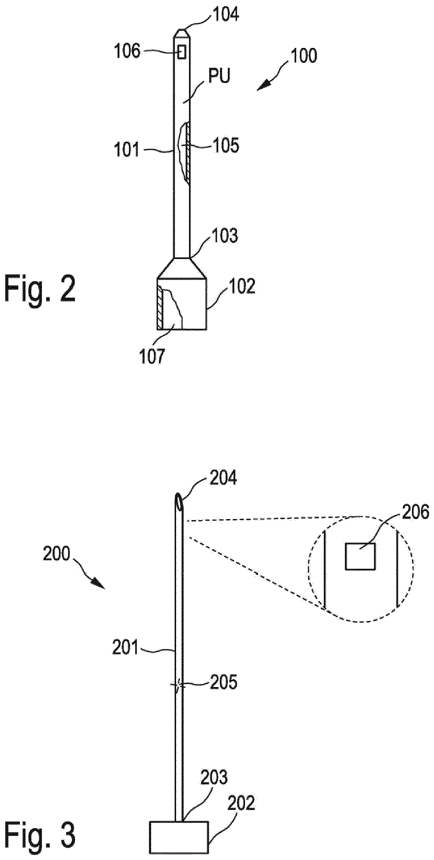

Das Kapillar 100 weist ein Kapillarrohr 101 und einen Kapillaransatz 102 auf (siehe Fig. 2). Der Kapillaransatz 102 ist an ein proximales Ende 103 des Kapillarrohrs 101 angefügt. Das Kapillarrohr 101 ist zwischen dem proximalen Ende 103 und einem distalen Ende 104 längserstreckt. Das Kapillar 100, im Speziellen das Kapillarrohr 101, weist ein Lumen 105 auf. Das Lumen 105 ist proximodistal durchgängig. Das Kapillar 100 kann auch als Kurzkatheter bezeichnet werden.

Bei der gezeigten Ausführungsform ist das Kapillarrohr 101 biegeflexibel ausgebildet. Das Kapillarrohr 101 ist vorliegend aus einem biegeflexiblen Kunststoffmaterial gefertigt. Im Speziellen ist das Kapillarrohr 101 aus Polyurethan PU gefertigt.

Die Kanüle 200 weist ein Kanülenrohr 201 und einen Kanülenansatz 202 auf. Der Kanülenansatz 202 ist an ein proximales Ende 203 des Kanülenrohrs 201 angefügt. Das Kanülenrohr 201 ist zwischen dem proximalen Ende 203 und einem distalen Ende 204 längserstreckt. Das distale Ende 204 weist ein nicht näher bezeichnetes geschliffenes Kanülenauge auf. Die Kanüle 200, im Speziellen das Kanülenrohr 201, weist ein Lumen 205 auf. Das Lumen 205 ist proximodistal durchgängig. Das Lumen 205 mündet einends in das nicht näher bezeichnete Kanülenauge. Das Kanülenrohr 201 ist in das Kapillarrohr 101 einschiebbar. Zudem sind der Kanülenansatz 202 und der Kapillaransatz 102 lösbar miteinander verbindbar. In Fig. 1 ist ein eingeschobener und verbundener Zustand gezeigt. Zum Einschieben des Kanülenrohrs 201 wird dessen distales Endes 204 durch den Kapillaransatz 102 in das Kapillarrohr 101, genauer: dessen Lumen 105, distal eingeschoben. In eingeschobenem Zustand ragt das distale Ende 204 des Kanülenrohrs 201 über das distale Ende 104 des Kapillarrohrs 101 hinaus (siehe Fig. 1). Das Kapillarrohr 101 und das Kanülenrohr 201 sind maßlich aufeinander abgestimmt. Insbesondere ist ein Innendurchmesser des Kapillarrohrs 101 größer als ein Außendurchmesser des Kanülenrohrs 201. Der Innendurchmesser und der Außendurchmesser sind ohne Bezugszeichen. Zudem ist eine axiale Länge des Kanülenrohrs 201 größer als eine axiale Länge des Kapillarrohrs 101. Die beiden axialen Längen sind ebenfalls ohne Bezugszeichen. Die lösbare Befestigung zwischen dem Kapillaransatz 102 und dem Kanülenansatz 202 ist form- und/oder kraftschlüssig. Der Kapillaransatz 102 und der Kanülenansatz 202 sind zur lösbaren Befestigung eingerichtet. Die lösbare Befestigung ist beispielsweise eine Schraub-, Steck- und/oder Klemmverbindung. Der Kapillaransatz 102 und der Kanülenansatz 202 sind wenigstens abschnittsweise dementsprechend komplementär ausgebildet.

Der Katheterschlauch 300 ist zwischen einem proximalen Ende 301 und einem distalen Ende 302 längserstreckt. Es versteht sich, dass der Katheterschlauch 300 ein Lumen aufweist. Das Lumen ist mit dem Bezugszeichen 303 belegt und proximodistal durchgängig. Der Katheterschlauch 300 ist in distaler Richtung durch das Kapillarrohr 101 vorschiebbar. Zu diesem Zweck sind der Katheterschlauch 300 und das Kapillar 100 maßlich aufeinander abgestimmt. Insbesondere ist ein Außendurchmesser des Katheterschlauchs 300 kleiner als ein Innendurchmesser des Kapillarrohrs 101. Die besagten Durchmesser sind wiederum ohne Bezugszeichen.

Bei der gezeigten Ausführungsform weist der Katheterschlauch 300 einen herausziehbaren Mandrin 305 auf. Der Mandrin 305 ragt in der in Fig. 1 gezeigten Konfiguration aus dem proximalen Ende 301 des Katheterschlauchs 300. Der Mandrin 305 ist innerhalb des Lumens 303 längserstreckt. Der Mandrin 305 wirkt einem ungewollten Knicken des Katheterschlauchs 300 entgegen. Hierdurch wird das Einführen und Vorschieben des Katheterschlauchs erleichtert. Der Mandrin 305 wirkt mechanisch stabilisierend. Bei einer in den Figuren nicht gezeigten Ausführungsform weist der Katheterschlauch keinen Mandrin auf.

Die Katheterkupplung 400 ist zur Befestigung an dem proximalen Ende 301 des Katheterschlauchs 300 eingerichtet. Die Art und Weise der Befestigung ist bei unterschiedlichen Ausgestaltungen unterschiedlich ausgeführt. Bei einer Ausgestaltung ist die Katheterkupplung mit dem proximalen Ende 301 zusammengesteckt. Alternativ oder zusätzlich kann eine Klemmung oder sonstige geeignete Fixierung ausgebildet sein. Die spezifischen gegenständlichen und funktionellen Merkmale der Katheterkupplung 400 sind im Hinblick auf die vorliegende Erfindung nicht wesentlich. Eine für das Katheterset 1 grundsätzlich geeignete Katheterkupplung wird seitens der Anmelderin unter der Bezeichnung Perifix^{®} am Markt angeboten.

Die Fluidzuleitung 500 weist einen Schlauch 501 und wenigstens einen Fluidkonnektor 502 auf. Vorliegend ist ein weiterer Fluidkonnektor 505 vorhanden. Die beiden Fluidkonnektoren 502, 505 werden nachfolgend auch als proximaler Fluidkonnektor 505 und distaler Fluidkonnektor 502 bezeichnet. Der proximale Fluidkonnektor 505 ist an ein proximales Ende 503 des Schlauchs 501 angefügt. Der distale Fluidkonnektor 502 ist an ein distales Ende 504 des Schlauchs 501 angefügt. Der Schlauch 501 ist zwischen dem proximalen Ende 503 und dem distalen Ende 504 längserstreckt. Die Fluidzuleitung 500 weist ein Lumen 506 auf. Das Lumen 506 ist proximodistal durchgängig. Der distale Fluidkonnektor 502 ist zur lösbaren Verbindung mit dem Kapillaransatz 102 eingerichtet. Der distale Fluidkonnektor 502 und der Kapillaransatz 102 sind dementsprechend aufeinander abgestimmt und/oder eingerichtet. Die lösbare Verbindung ist bei unterschiedlichen Ausgestaltungen des Kathetersets 1 unterschiedlich ausgeführt. Beispielsweise kann eine Steck-, Klemm- und/oder Schraubverbindung zwischen dem Kapillaransatz 102 und dem distalen Fluidkonnektor 502 vorgesehen sein. Der distale Fluidkonnektor 502 und der Kapillaransatz 102 sind wenigstens abschnittsweise dementsprechend komplementär ausgebildet. Die Fluidzuleitung 500 kann auch als Verlängerungsleitung bezeichnet werden (englisch: extension line).

Die Fixierkomponente 600 ist zum Ausbilden der dritten Katheteranordnung 30 erforderlich (Fig. 6). Die Fixierkomponente 600 ist auf noch näher beschriebene Weise an dem Kapillaransatz 102 befestigbar. Die Fixierkomponente 600 dient einer axialen Fixierung des in das Kapillarrohr 101 eingeschobenen Katheterschlauchs 300 (siehe Fig. 6). Die Fixierkomponente 600 ist dementsprechend eingerichtet und wirkt hierfür zum einen mit dem Kapillar 100, genauer dem Kapillaransatz 102, und zum anderen mit dem Katheterschlauch 300 zusammen.

Bei der gezeigten Ausführungsform weist das Katheterset 1 mehrere zur ultraschallbasierten Ortung eingerichtete Sensoren 106, 206, 306 auf. Die Sensoren 106, 206, 306 können auch als erster Sensor 106, zweiter Sensor 206 und dritter Sensor 306 bezeichnet werden. Der erste Sensor 106 ist am distalen Ende des Kapillarrohrs 101 angeordnet. Vorliegend ist der erste Sensor 106 in das Kapillarrohr 101 eingebettet und/oder integriert. Der zweite Sensor 206 ist an dem distalen Ende 204 des Kanülenrohrs 201 angeordnet. Vorliegend ist der zweite Sensor 206 in eine nicht näher bezeichnete Wandung des Kanülenrohrs 201 eingebettet und/oder integriert. Der dritte Sensor 306 ist an dem distalen Ende 302 des Katheterschlauchs 300 angeordnet. Vorliegend ist der dritte Sensor 306 in eine nicht näher bezeichnete Schlauchwandung des Katheterschlauchs 300 eingebettet und/oder integriert. Die Sensoren 106, 206, 306 erlauben eine ultraschallbasierte Navigation und/oder Ortung. Hierdurch kann die betreffende Komponente des Kathetersets 1 beim Anlegen an den Patienten besonders präzise positioniert werden. Alternativ oder zusätzlich kann eine ultraschallbasierte Positionskontrolle nach erfolgter Anlage durchgeführt werden. Sensoren zur ultraschallbasierten Ortung sind dem Fachmann grundsätzlich bekannt. Weitere spezifische gegenständliche und/oder funktionelle Merkmale der Sensoren 106, 206, 306 sind im Hinblick auf die vorliegende Erfindung nicht wesentlich. Weitere Erläuterungen zu den Sensoren 106, 206, 306 sind daher entbehrlich.

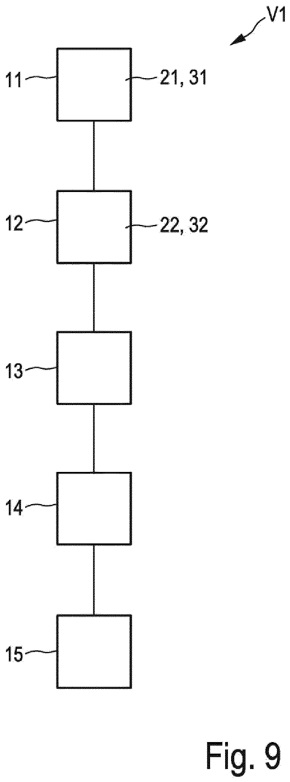

Die Fig. 4, 5, 6 zeigen die bereits erläuterten unterschiedlichen Katheteranordnungen 10, 20, 30. Im Einzelnen:
Die erste Katheteranordnung 10 weist den Katheterschlauch 300 und die Katheterkupplung 400 auf. In dieser ersten Katheterlage erfolgt die Verabreichung des Anästhetikums über den Katheterschlauch 300. Das Anästhetikum wird hierfür mittels einer proximal an die Katheterkupplung 400 anschließbaren Fluidleitungskomponente, beispielsweise einem Schlauch oder einer Spritze, über die Katheterkupplung 400 in das Lumen 303 und von dort über das distale Ende 302 an den zu blockierenden Nerv abgegeben. Das Katheterset 10 kann gemäß dem schematisch in Fig. 9 gezeigten Verfahren unter Verwendung des Kathetersets 1 ausgebildet werden. Das in Fig. 9 schematisch verdeutlichte Verfahren wird nachfolgend auch als erstes Verfahren V1 bezeichnet. Das erste Verfahren V1 weist folgende Schritte auf: In einem ersten Schritt 11 wird das Katheterset 1 bereitgestellt. In einem zweiten Schritt 12 wird die Kanüle - ausgehend von dem in Fig. 1 gezeigten mit dem Kapillar 100 zusammengefügten Zustand - entfernt. Hierfür wird der Kanülenansatz 202 von dem Kapillaransatz 102 gelöst. Weiter wird das Kanülenrohr 201 in proximaler Richtung aus dem Kapillarrohr 101 herausgezogen. In einem dritten Schritt 13 wird der Katheterschlauch 300 angebracht. Hierfür wird das distale Ende 302 des Katheterschlauchs 300 durch den Kapillaransatz 102 in das Kapillarrohr 101, genauer das Lumen 105, eingeführt und proximal vorgeschoben. In einem vierten Schritt 14 wird das Kapillar 100 entfernt. Hierfür wird das Kapillarrohr 101 gemeinsam mit dem Kapillaransatz 102 in proximaler Richtung von dem Katheterschlauch 300 abgezogen. In einem fünften Schritt 15 wird die Katheterkupplung 400 an dem proximalen Ende 301 des Katheterschlauchs 300 befestigt. Vor dem Befestigen der Katheterkupplung 400 wird vorliegend der Mandrin 305 in proximaler Richtung aus dem Katheterschlauch 300 herausgezogen.

Die zweite Katheteranordnung 20 (siehe Fig. 5) weist das Kapillar 100 und die Fluidzuleitung 500 auf. Der (distale) Fluidkonnektor 502 ist an dem Kapillaransatz 102 befestigt. Die Verabreichung des Anästhetikums erfolgt hier über das Kapillar 100. Das Anästhetikum wird über die Fluidzuleitung 500 dem Kapillar 100 zugeleitet. Das Anästhetikum gelangt hierbei über das Lumen 506 des Schlauchs 501 in das Lumen 105 des Kapillarrohrs 101 und von dort über das distale Ende 104 an den zu blockierenden Nerv.

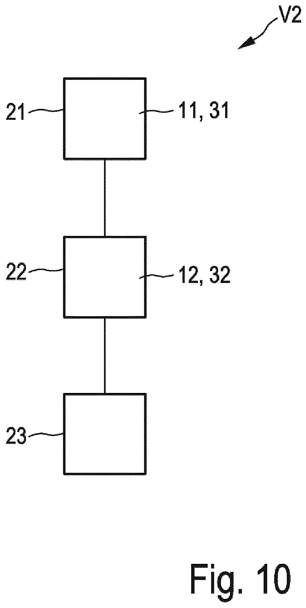

Fig. 10 zeigt schematisch ein zweites Verfahren V2 zum Ausbilden der zweiten Katheteranordnung 20 unter Verwendung des Kathetersets 1. Das zweite Verfahren V2 weist einen ersten Schritt 21 und einen zweiten Schritt 22 auf. Der erste Schritt 21 und der zweite Schritt 22 sind jeweils identisch mit dem betreffenden Schritt des ersten Verfahrens V1. Zur Vermeidung von Wiederholungen wird auf das dort Gesagte verwiesen und ausdrücklich Bezug genommen. Das zweite Verfahren V2 weist einen dritten Schritt 23 auf. In diesem dritten Schritt wird die Fluidzuleitung 500 angebracht. Hierfür wird der distale Fluidkonnektor 502 an dem Kapillaransatz 102 befestigt.

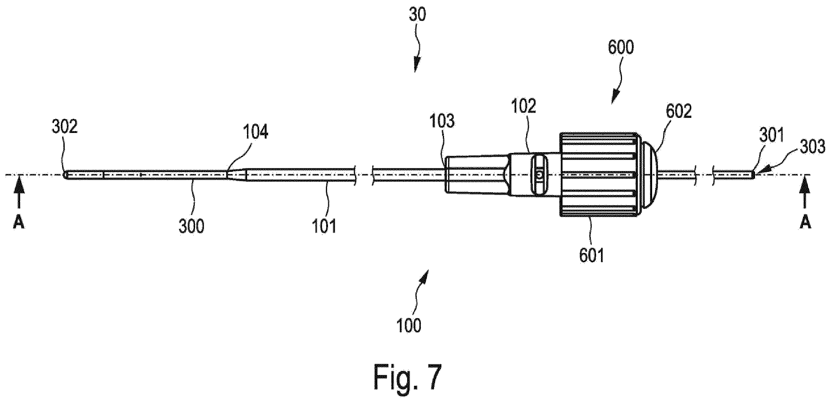

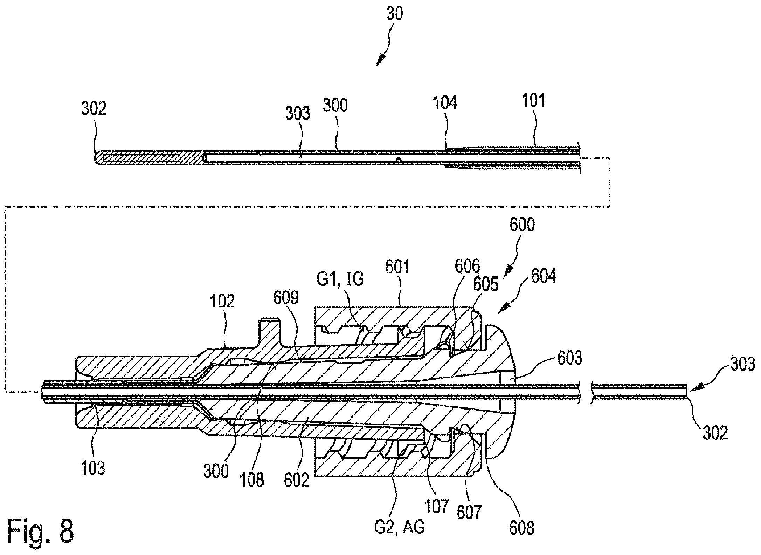

Die dritte Katheteranordnung 30 (siehe insbesondere Fig. 6) weist das Kapillar 100, den Katheterschlauch 300 und die Fixierkomponente 600 auf. Zudem weist die dritte Katheteranordnung 30 vorliegend die Katheterkupplung 400 auf. Der Katheterschlauch 300 ist durch den Kapillaransatz 102 in das Kapillarrohr 101 eingeschoben. Dabei ragt das distale Ende 302 des Katheterschlauchs 300 über das distale Ende 104 des Kapillarrohrs 101 hinaus. In der anhand Fig. 6 gezeigten Konfiguration ist der Katheterschlauch 300 mittels der Fixierkomponente 600 in seiner axialen Beweglichkeit relativ zu dem Kapillar 100 fixiert. Auf den spezifischen Aufbau und die weitere Funktion der Fixierkomponente wird anhand der Fig. 7 und 8 noch näher Bezug genommen.

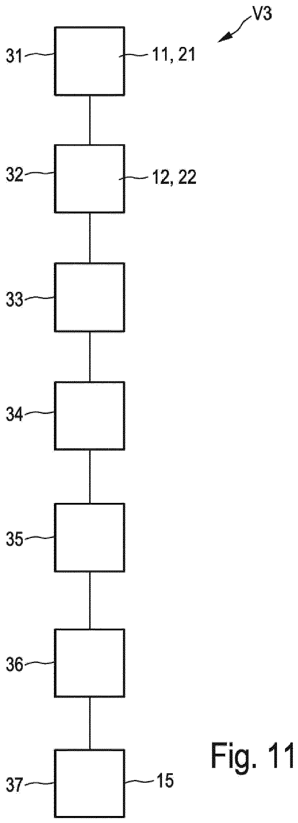

Zunächst wird unter Bezugnahme auf Fig. 11 ein drittes Verfahren V3 zum Ausbilden der dritten Katheteranordnung 30 unter Verwendung des Kathetersets 1 eingegangen. Das dritte Verfahren V3 weist einen ersten Schritt 31 und einen zweiten Schritt 32 auf. Die beiden Schritte 31, 32 sind identisch zu dem jeweiligen Schritt des ersten Verfahrens V1 und des zweiten Verfahrens V2. Hinsichtlich des ersten Schritts 31 wird zur Vermeidung von Wiederholungen auf die Erläuterungen zu dem ersten Schritt 11 des ersten Verfahrens V1 verwiesen und ausdrücklich Bezug genommen. Dies gilt mutatis mutandis auch für den zweiten Schritt 32. Das dritte Verfahren V3 weist einen dritten Schritt 33 auf. In dem dritten Schritt 33 wird die Fixierkomponente 600 an dem Kapillar 100 angebracht. Hierfür wird die Fixierkomponente 600 an dem Kapillareinsatz 102 befestigt. Dies auf noch näher beschriebene Weise. In einem vierten Schritt 34 wird der Katheterschlauch 300 angebracht. Hierfür wird dessen distales Ende 302 durch die Fixierkomponente 600 und den Kapillaransatz 102 in das Kapillarrohr 101 eingeführt und distal entlang des Kapillarrohrs 101 vorgeschoben. In einem fünften Schritt 35 wird der Katheterschlauch 300 relativ zu dem Kapillar 100 und damit auch der Fixierkomponente 600 positioniert. Hierfür wird das distale Ende 302 des Katheterschlauchs 300 bis zu einer gewünschten Position in Bezug auf das distale Ende 104 des Kapillarrohrs 101 vorgeschoben und/oder zurückgezogen. In einem sechsten Schritt 36 wird der Katheterschlauch 300 fixiert. Hierfür wird die Fixierkomponente 600 auf noch näher beschriebene Weise betätigt und der Katheterschlauch 300 wird unter Einwirkung der Fixierkomponente relativ zu dem Kapillar 100 axial festgelegt. Vorliegend wird in einem siebten Schritt 37 die Katheterkupplung 400 an dem proximale Ende 301 des Katheterschlauchs 300 angebracht. Der Schritt 37 korrespondiert mit dem Schritt 15 des Verfahrens V1.

Die Fig. 7 und 8 zeigen eine alternative, detailliertere Darstellung der dritten Katheteranordnung 30. Die besagten Figuren lassen weitere Merkmale der Fixierkomponente 600 erkennen. Die Katheterkupplung 400 ist in den Fig. 7 und 8 nicht dargestellt.

Die Fixierkomponente 600 weist bei der gezeigten Ausführungsform ein Betätigungselement 601 und ein Klemmelement 602 auf.

Das Betätigungselement 601 ist relativ zu dem Kapillaransatz 102 beweglich an dem Kapillaransatz 102 gelagert und kraft- und/oder bewegungsübertragend mit dem Klemmelement 602 wirkverbunden.

Das Klemmelement 602 ist in einer Aufnahmeaussparung 107 (siehe auch Fig. 2) des Kapillaransatzes 102 aufgenommen. Das Klemmelement 602 weist ein koaxial zu dem Kapillarrohr 102 ausgerichtetes Lumen 603 auf. Das Lumen 603 ist proximodistal durchgängig zwischen einem proximalen Ende und einem distalen Ende des Klemmelements 602 längserstreckt. Die besagten Enden des Klemmelements 602 sind ohne Bezugszeichen. Das Lumen 603 ist zur Aufnahme des Katheterschlauchs 300 eingerichtet. In der gemäß den Fig. 6 bis 8 gezeigten Assemblierung der Komponenten ist der Katheterschlauch 300 durch das Lumen 603 längserstreckt. Das Klemmelement 602 ist formnachgiebig. Zu diesem Zweck ist das Klemmelement 602 vorliegend aus einem elastomeren Kunststoffmaterial gefertigt.

Das Klemmelement 602 ist mittels einer Bewegung des Betätigungselements 601 elastisch deformierbar zwischen einer Klemmkonfiguration (Fig. 8) und einer Freigabekonfiguration. Die Freigabekonfiguration ist in den Figuren nicht gesondert dargestellt. In der Klemmkonfiguration ist das Klemmelement 602 unter Einwirkung des Betätigungselements 601 einerseits und andererseits des Kapillaransatzes 102 derart elastisch deformiert, dass das Lumen 603 radial verengt und hierdurch die axiale Beweglichkeit des Katheterschlauchs 300 reibschlüssig fixiert ist. In der Freigabekonfiguration ist die elastische Deformation weniger stark ausgeprägt oder nicht vorhanden. Der Reibschluss ist dann so weit aufgehoben, dass die axiale Beweglichkeit des Katheterschlauchs 300 in dem Lumen 603 freigegeben ist.

Bei der gezeigten Ausführungsform ist das Betätigungselement 601 relativ zu dem Kapillaransatz 102 schraubbeweglich. Das Betätigungselement 601 weist einen Gewindeabschnitt G1 auf. Der Kapillaransatz 102 weist einen hierzu komplementären Gewindeabschnitt G2 auf. Bei der gezeigten Ausführungsform ist der Gewindeabschnitt G1 ein Innengewinde IG. Der komplementäre Gewindeabschnitt G2 ist ein Außengewinde AG. Das Außengewinde AG ist an einem proximalen Ende 107 des Kapillaransatzes 102 ausgebildet. Die beiden Gewindeabschnitte G1, G2 sind koaxial zu dem Lumen 603 orientiert. Bei einer Schraubbewegung im Uhrzeigersinn bewegt sich das Betätigungselement 601 relativ zu dem Kapillaransatz 102 distal. Eine Schraubbewegung entgegen dem Uhrzeigersinn führt zu einer proximalen Verlagerung.

Die kraft- und/oder bewegungsübertragende Wirkverbindung zwischen dem Betätigungselement 601 und dem Klemmelement 602 ist vorliegend axial formschlüssig. In Umfangsrichtung wirken das Betätigungselement 601 und das Klemmelement 602 gleitbeweglich zusammen. Zu diesem Zweck weist das Klemmelement 602 einen proximal angeordneten Lagerabschnitt 604 auf. Der Lagerabschnitt 604 weist eine Gleitfläche 605 auf. Die Gleitfläche 605 wird distal von einer Anlaufschulter 606 begrenzt. Das Betätigungselement 601 weist eine nicht näher bezeichnete Bohrung auf. Eine Innenumfangsfläche 607 der Bohrung ist in Umfangsrichtung gleitbeweglich auf der Gleitfläche 605 gelagert. Der Rand der Bohrung läuft distal an die Anlaufschulter 606 an. Der Lagerabschnitt 604 weist bei der gezeigten Ausführungsform eine weitere Anlaufschulter 608 auf. Die weitere Anlaufschulter 608 kann auch als proximale Anlaufschulter bezeichnet werden. Die proximale Anlaufschulter 608 wirkt bei einer proximalen Verlagerung des Betätigungselements 601 formschlüssig mit dem Rand der Bohrung zusammen.

Die Aufnahmeaussparung 107 weist eine Innenkonusfläche 108 auf. Das Klemmelement 602 weist eine wenigstens abschnittsweise komplementäre Außenkonusfläche 609 auf.

In der Klemmkonfiguration ist das Klemmelement 602 distal in die Aufnahmeaussparung 107 hineingepresst. Hierdurch werden die beiden Konusflächen 108, 609 gegeneinander angestellt.

Dies führt zu der besagten elastischen Deformation des Klemmelements 602 und der hieraus resultierenden radialen Verengung des Lumens 603.

Es versteht sich, dass die anhand der Fig. 7 und 8 erläuterten spezifischen gegenständlichen und funktionellen Merkmale der Fixierkomponente 600 im Hinblick auf die vorliegende Erfindung nicht wesentlich sind. Die besagten Merkmale sind jedoch als vorteilhaft zu erachten.

## Patentansprüche

1. Katheterset (1) zum Ausbilden unterschiedlicher Katheteranordnungen (10, 20, 30) für eine periphere Regionalanästhesie, aufweisend
ein Kapillar (100) mit einem Kapillarrohr (101) und einem Kapillaransatz (102), welcher an ein proximales Ende (103) des Kapillarrohrs (101) angefügt ist,
eine Kanüle (200) mit einem Kanülenrohr (201), welches in das Kapillarrohr (101) eingeschoben oder einschiebbar ist, und mit einem Kanülenansatz (202), welcher an ein proximales Ende (203) des Kanülenrohrs (201) angefügt und mit dem Kapillaransatz (102) lösbar verbunden oder verbindbar ist,
einen Katheterschlauch (300), welcher in distaler Richtung durch das Kapillarrohr (101) vorschiebbar ist,
eine Katheterkupplung (400), welche zur Befestigung an einem proximalen Ende (301) des Katheterschlauchs (300) eingerichtet ist,
und eine Fluidzuleitung (500) mit einem Schlauch (501) und wenigstens einem Fluidkonnektor (502), welcher an ein distales Ende (504) des Schlauchs (501) angefügt und mit dem Kapillaransatz (102) lösbar verbindbar ist,
wobei das Katheterset (1) zum wahlweisen Ausbilden wenigstens einer ersten Katheteranordnung (10) und einer zweiten Katheteranordnung (20) eingerichtet ist,
wobei die erste Katheteranordnung (10) den Katheterschlauch (300) und die am proximalen Ende (301) desselben befestigte Katheterkupplung (400) aufweist und zur Abgabe eines Anästhetikums über den Katheterschlauch (300) eingerichtet ist,
und wobei die zweite Katheteranordnung (20) das Kapillar (100) und die Fluidzuleitung (500), welche mittels des Fluidkonnektors (502) mit dem Kapillaransatz (102) verbunden ist, aufweist und zur Abgabe des Anästhetikums über das Kapillarrohr (101) eingerichtet ist.

2. Katheterset (1) nach Anspruch 1, weiter aufweisend
eine Fixierkomponente (600), welche an dem Kapillaransatz (102) befestigbar und zur axialen Fixierung des in das Kapillarrohr einschiebbaren Katheterschlauchs (300) an dem Kapillar (100) eingerichtet ist,
wobei das Katheterset (1) zum wahlweisen Ausbilden einer dritten Katheteranordnung (30) eingerichtet ist, welche das Kapillar (100), den Katheterschlauch (300), die Fixierkomponente (600) und die am proximalen Ende (301) des Katheterschlauchs (300) befestigte Katheterkupplung (400) aufweist, wobei die Fixierkomponente (600) an dem Kapillaransatz (102) befestigt ist und der Katheterschlauch (300) zur Abgabe des Anästhetikums in distaler Richtung durch das Kapillarrohr (101) vorgeschoben sowie mittels der Fixierkomponente (600) relativ zu dem Kapillar (100) axial fixiert ist.

3. Katheterset (1) nach Anspruch 1 oder 2, **dadurch gekennzeichnet, dass** das Kapillarrohr (101) biegeflexibel ausgebildet ist.

4. Katheterset (1) nach Anspruch 3, **dadurch gekennzeichnet, dass** das Kapillarrohr (101) aus Polyurethan (PU) gefertigt ist.

5. Katheterset (1) nach einem der vorhergehenden Ansprüche, **dadurch gekennzeichnet, dass** der Katheterschlauch (300) einen herausziehbaren Mandrin (305) aufweist.

6. Katheterset (1) nach einem der vorhergehenden Ansprüche, **dadurch gekennzeichnet, dass** mehrere zur ultraschallbasierten Ortung eingerichtete Sensoren (106, 206, 306) vorhanden und an unterschiedlichen Komponenten des Kathetersets (1) angebracht sind.

7. Katheterset (1) nach Anspruch 6, **dadurch gekennzeichnet, dass** ein erster Sensor (101) in das distale Ende des Kapillarrohrs (101) integriert ist und/oder ein zweiter Sensor (206) in das distale Ende (204) des Kanülenrohrs (201) integriert ist und/oder ein dritter Sensor (306) in ein distales Ende (302) des Katheterschlauchs (300) integriert ist.

8. Katheterset (1) nach einem der Ansprüche 2 bis 7, **dadurch gekennzeichnet, dass**
die Fixierkomponente (600) ein Betätigungselement (601) und ein formnachgiebiges Klemmelement (602) aufweist,
wobei das Klemmelement (602) in eine Aufnahmeaussparung (107) des Kapillaransatzes (102) aufnehmbar oder aufgenommen ist und ein - in aufgenommenem Zustand - koaxial zu dem Kapillarrohr (101) ausgerichtetes Lumen (603) aufweist, in welches der Katheterschlauch (300) distal einschiebbar ist,
wobei das Betätigungselement (601) relativ zu dem Kapillaransatz (102) beweglich an dem Kapillaransatz (102) lagerbar oder gelagert ist und kraft- und/oder bewegungsübertragend mit dem Klemmelement (602) wirkverbunden ist,
und wobei das Klemmelement (602) mittels einer Bewegung des Betätigungselements (601) elastisch deformierbar ist zwischen einer Klemmkonfiguration, in welcher das Lumen (603) radial verengt und eine axiale Beweglichkeit des Katheterschlauchs (300) reibschlüssig fixiert ist, und einer Freigabekonfiguration, in welcher der Reibschluss aufgehoben und die axiale Beweglichkeit des Katheterschlauchs (300) freigegeben ist.

9. Verfahren (V1) zum Ausbilden einer ersten Katheteranordnung (10), aufweisend die Schritte:
Bereitstellen (11) eines Kathetersets (1) nach einem der Ansprüche 1 bis 8;
Entfernen (12) der Kanüle (200), wobei der Kanülenansatz (202) von dem Kapillaransatz (102) gelöst und das in dem Kapillarrohr (101) vorgeschobene Kanülenrohr (201) in proximaler Richtung aus dem Kapillarrohr (101) herausgezogen wird;
Anbringen (13) des Katheterschlauchs (300), wobei dessen distales Ende (302) in den Kapillaransatz (102) eingeführt und in distaler Richtung entlang des Kapillarrohrs (101) vorgeschoben wird;
Entfernen (14) des Kapillars (100), wobei das Kapillarrohr (101) in proximaler Richtung von dem Katheterschlauch (300) abgezogen wird;
Befestigen (15) der Katheterkupplung (400) an dem proximalen Ende (301) des Katheterschlauchs (300).

10. Verfahren (V2) zum Ausbilden einer zweiten Katheteranordnung (20), aufweisend die Schritte:
Bereitstellen (21) eines Kathetersets (1) nach einem der Ansprüche 1 bis 8;
Entfernen (22) der Kanüle, wobei der Kanülenansatz (202) von dem Kapillaransatz (102) gelöst und das in dem Kapillarrohr (101) vorgeschobene Kanülenrohr (201) in proximaler Richtung aus dem Kapillarrohr (101) herausgezogen wird;
Anbringen (23) der Fluidzuleitung (500), wobei deren Fluidkonnektor (502) mit dem Kapillaransatz (102) verbunden wird.

11. Verfahren (V3) zum Ausbilden einer dritten Katheteranordnung (30), aufweisend die Schritte:
Bereitstellen (31) eines Kathetersets (1) nach einem der Ansprüche 2 bis 8;
Entfernen (32) der Kanüle, wobei der Kanülenansatz (202) von dem Kapillaransatz (102) gelöst und das in dem Kapillarrohr (101) vorgeschobene Kanülenrohr (201) in proximaler Richtung aus dem Kapillarrohr (101) herausgezogen wird;
Anbringen (33) der Fixierkomponente (600) an dem Kapillar (100), wobei die Fixierkomponente (600) an dem Kapillaransatz (102) befestigt wird;
Anbringen (34) des Katheterschlauchs (300), wobei dessen distales Ende (302) in den Kapillaransatz (102) eingeführt und in distaler Richtung entlang des Kapillarrohrs (101) vorgeschoben wird;
Positionieren (35) des Katheterschlauchs (300), wobei dessen distales Ende (302) bis zu einer gewünschten Position in Bezug auf das distale Ende (104) des Kapillarrohrs (101) vorgeschoben wird;
Fixieren (36) des Katheterschlauchs (300), wobei die Fixierkomponente (600) betätigt und der Katheterschlauch (300) unter Einwirkung der Fixierkomponente (600) relativ zu dem Kapillar (100) axial fixiert wird;
Befestigen (37) der Katheterkupplung (400) an dem proximalen Ende (301) des Katheterschlauchs (300).
